# EUROPEAN PATENT SPECIFICATION

(11) **EP 3 269 404 B1**
(45) Date of publication and mention of the grant of the patent: **14.10.2020**
(21) Application number: 16761884.2
(22) Date of filing: 19.01.2016
(51) Int. Cl.: A61M 3/02, A61M 1/00

(54) **METHOD AND DEVICE FOR HEALING WOUND**
VERFAHREN UND VORRICHTUNG ZUR WUNDHEILUNG
PROCÉDÉ ET DISPOSITIF DE CICATRISATION DE PLAIE

(30) Priority: 10.03.2015 KR 20150033105
(43) Date of publication of application: 17.01.2018
(73) Proprietor: CG Bio Co., Ltd., Seongnam-si, Gyeonggi-do 13211 (KR)
(72) Inventor: RYU, Hyun Seung, Seongnam-si Gyeonggi-do 13611 (KR); HONG, Joon Pio, Seoul (KR); SEO, Jun Hyuk, Hanam-si, Gyeonggi-do (KR); PARK, Hee Jun, Seoul 06093 (KR); HONG, Soon Gee, Jeonju-si Jeollabuk-do 55124 (KR); JEON, Kang Jin, Seongnam-si Gyeonggi-do 13197 (KR); KIM, Hoon, Seongnam-si Gyeonggi-do 13565 (KR)
(74) Representative: Eisenführ Speiser
(86) International application number: PCT/KR2016/000524
(87) International publication number: WO 2016/143997

(56) References cited:
- WO-A1-2013/123022
- JP-A- 2007 534 407
- JP-A- 2010 525 873
- KR-A- 20110 083 693
- KR-A- 20110 116 141
- US-A1- 2003 021 775
- US-A1- 2013 211 318

## Description

### BACKGROUND

### 1. Field of the Invention

The present disclosure relates to a wound treatment method and apparatus, and more particularly, to a wound treatment method and apparatus capable of promoting the treatment of a wound.

### 2. Description of the Related Art

A wound treatment apparatus according to WO 2013/123022 A1 combines an internal negative pressure (vacuum) pump and an internal positive pressure (compressor) pump connectable to an external oxygen supply for providing both negative pressure wound therapy and hyperbaric oxygen wound therapy to a wound site. The apparatus also includes a user interface operatively connected to an electronic controller that monitors and actuates the vacuum and compressor pumps. The user interface and controller enables the apparatus to provide multiple modes of operation and the ability to selectively change between negative pressure therapy operational modes and hyperbaric oxygen operational modes.

US 2013/211318 A1 discloses a method for determining a suitable volume of instillation fluid for instillation therapy of wounds.

A wound treatment method using negative pressure is commonly used in hospitals to promote the treatment of wounds. In the wound treatment method using negative pressure, a curer places a foam dressing at a wound site.

Then, the curer seals the wound site by attaching a film dressing onto the skin adjacent to the wound site. Once the wound site is sealed, the curer places one end of a drain tube in communication with the sealed space between the film dressing and the wound site.

Thereafter, the curer supplies negative pressure to the sealed space between the film dressing and the wound site by driving a negative pressure generation unit, which is placed in communication with the other end of the drain tube. As a result, exudate from the wound site is absorbed by the foam dressing, and the absorbed exudate is discharged from the sealed space between the film dressing and the wound site through the drain tube.

Since the exudate is removed from the wound site, the formation of granulation tissue in the wound site is promoted, and thus, the treatment of the wound site is promoted. However, since the wound site is sealed, the wound site is at the risk of getting infected with pathogens present therein and becoming worse, and research is underway to address this and other issues.

### SUMMARY

Exemplary embodiments of the present disclosure provide a wound treatment method and apparatus capable of promoting the treatment of a wound.

However, exemplary embodiments of the present disclosure are not restricted to those set forth herein. The above and other exemplary embodiments of the present disclosure will become more apparent to one of ordinary skill in the art to which the present disclosure pertains by referencing the detailed description of the present disclosure given below.

According to an exemplary embodiment of the present disclosure, a wound treatment apparatus is provided as defined in claim 1. Insofar as the term invention or embodiment is used in the following, or features are presented as being optional, this should be interpreted in such a way that the only protection sought is that of the invention claimed.

In some embodiments of the present invention, wherein once the pressure in the sealed space reaches the target negative pressure level, the controller controls the negative pressure generation unit to maintain the pressure in the sealed space at the target negative pressure level for a first setting period.

In some embodiments of the present invention, wherein the controller controls the negative pressure generation unit to stop the supply of the negative pressure for a second setting period, which follows the first setting period, and controls the irrigator to inject the medication at a time when the supply of the negative pressure is stopped.

In some embodiments of the present invention, wherein the controller controls the irrigator to stop the injection of the medication within the second setting period.

In some embodiments of the present invention, wherein the controller controls the irrigator to stop the injection of the medication after the second setting period.

In some embodiments of the present invention, wherein the first setting period is longer than the second setting period.

In some embodiments of the present invention, further comprising: an oxygen supplier supplying oxygen to the medication, which is to be injected into the wound site. The controller controls the irrigator to inject the medication at a volume less than a volume of the wound site.

According to the exemplary embodiments, the cycle of replacement of a foam dressing and a film dressing may be lengthened by discharging exudate from a wound site and injecting a medication into the wound site.

Pathogens may be removed from the wound site by discharging the medication from the wound site after irrigating the wound site with the medication.

The injection of the medication into the wound site, which is sealed, may be facilitated by stopping the supply of negative pressure to the wound site and at the same time, injecting the medication into the wound site.

Other features and aspects will be apparent from the following detailed description, the drawings, and the claims.

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic view illustrating a wound treatment apparatus according to an exemplary embodiment of the present disclosure.
FIG. 2 is a flowchart illustrating an operating method of the wound treatment apparatus of FIG. 1.
FIGS. 3 through 6 are schematic views illustrating how the wound treatment apparatus of FIG. 1 operates.
FIG. 7 is a graph showing variations in the pressure in a sealed space during an operation of the wound treatment apparatus of FIG. 1.

### DETAILED DESCRIPTION OF THE EMBODIMENTS

The present invention will now be described more fully hereinafter with reference to the accompanying drawings, in which preferred embodiments of the invention are shown. This invention may, however, be embodied in different forms and should not be construed as limited to the embodiments set forth herein. Rather, these embodiments are provided so that this disclosure will be thorough and complete, and will filly convey the scope of the invention to those skilled in the art. The same reference numbers indicate the same components throughout the specification. In the attached figures, the thickness of layers and regions is exaggerated for clarity.

It will also be understood that when a layer is referred to as being "on" another layer or substrate, it can be directly on the other layer or substrate, or intervening layers may also be present. In contrast, when an element is referred to as being "directly on" another element, there are no intervening elements present.

Spatially relative terms, such as "beneath," "below," "lower," "above," "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the exemplary term "below" can encompass both an orientation of above and below. The device may be otherwise oriented (rotated 90 degrees or at other orientations) and the spatially relative descriptors used herein interpreted accordingly.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

Unless defined otherwise, all technical and scientific terms used herein have the same meaning as commonly understood by one of ordinary skill in the art to which this invention belongs. It is noted that the use of any and all examples, or exemplary terms provided herein is intended merely to better illuminate the invention and is not a limitation on the scope of the invention unless otherwise specified. Further, unless defined otherwise, all terms defined in generally used dictionaries may not be overly interpreted.

The present invention will be described with reference to perspective views, cross-sectional views, and/or plan views, in which preferred embodiments of the invention are shown. Thus, the profile of an exemplary view may be modified according to manufacturing techniques and/or allowances. That is, the embodiments of the invention are not intended to limit the scope of the present invention but cover all changes and modifications that can be caused due to a change in manufacturing process. Thus, regions shown in the drawings are illustrated in schematic form and the shapes of the regions are presented simply by way of illustration and not as a limitation.

Exemplary embodiments of the present disclosure will hereinafter be described with reference to the accompanying drawings.

The term "negative pressure", as used herein, may be defined as pressure lower than the atmospheric pressure. Since the atmospheric pressure may differ from one region to another region, the term "negative pressure", as used herein, may be defined as pressure lower than the atmospheric pressure in the region where a wound treatment apparatus resides.

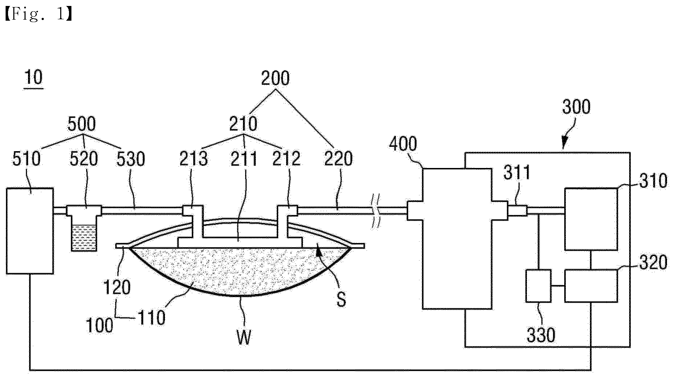

FIG. 1 is a schematic view illustrating a wound treatment apparatus according to an exemplary embodiment of the present disclosure.

Referring to FIG. 1, a wound treatment apparatus 10 includes a negative pressure output unit 100, a negative pressure delivery unit 200, a negative pressure generation unit 300, a canister 400, and a medication feeding unit 500.

As illustrated in FIG. 1, the negative pressure output unit 100 is placed at a wound site W and seals the wound site W. For convenience, the wound W will hereinafter be described, taking an open wound as an example, but the present disclosure is not limited thereto.

The negative pressure output unit 100 includes a foam dressing 110, which is placed at the wound site W, and a film dressing 120, which seals the wound site W.

The foam dressing 110 is placed at the wound site W and absorbs exudate from the wound site W using negative pressure.

To effectively discharge exudate from the wound W, the foam dressing 110 may be formed of polyurethane or polyether, but the present disclosure is not limited thereto. That is, any material capable of effectively discharging exudate may be used to form the foam dressing 110. For example, the foam dressing 110 may be formed of a polyurethane material, which is a hydrophobic material, in which case, the foam dressing 110 may easily discharge exudate.

The foam dressing 110 may include pores, which distribute negative pressure to the wound W and deliver a fluid (such as exudate) discharged from the wound W. For example, the foam dressing 110 may be formed to have an open-cell network structure capable of effectively discharging exudate.

The volume of the foam dressing 110 may be varied by negative pressure applied thereto by the negative pressure generation unit 300. For example, in response to negative pressure being applied to a sealed space S between a film dressing 120 and the wound site W, the foam dressing 110, the volume of the foam dressing 110 may be reduced so that the foam dressing 110 may be pressed against the surface of the wound site W. Then, in response to the supply of negative pressure to the sealed space S being terminated, the foam dressing 120 may return to its original state due to its restoring force. In this manner, the volume of the foam dressing 110 may be varied by negative pressure.

The film dressing 120 is attached onto the skin adjacent to the wound site W and seals the wound site W. As illustrated in FIG. 1, the film dressing 120 seals the wound site W except for part of a suction head 210.

In response to the film dressing 120 being attached onto the skin adjacent to the wound site W, the film dressing 120 may form the sealed space S together with the wound site W.

The film dressing 120 is formed of a material having elasticity. Accordingly, the film dressing 120 may not be torn or broken even when the pressure in the sealed space S changes.

The negative pressure delivery unit 200 delivers negative pressure generated by the negative pressure generation unit 300 to the negative pressure output unit 100.

As illustrated in FIG. 1, the negative pressure delivery unit 200 includes the suction head 210, which is connected to one side of the foam dressing 110, and a drain tube 220, which places the suction head 210 and the negative pressure generation unit 300 in communication with each other.

The suction head 210 guides, to the drain tube 220, the exudate absorbed into the foam dressing 110 due to negative pressure. In some exemplary embodiments, the suction head 220 may receive a medication from a connecting tube 530 of the medication feeding unit 500 and may inject the medication into the wound site W.

The suction head 210 includes a flange part 211, which is connected to one side of the foam dressing 110, a first connecting duct part 212, which is connected to the drain tube 220, and a second connecting duct part 213, which is connected to the first connecting duct part 212 and the connecting tube 530.

The flange part 211 forms a plurality of guide flow paths (not illustrated), which are connected to at least one of the first connecting duct part 212 and the second connecting duct part 213. The flange part 211 guides the exudate absorbed into the foam dressing 110 to the first connecting duct part 212 through the plurality of guide flow paths. Also, the flange part 211 evenly applies a medication supplied thereto from the medication feeding unit 500 through the second connecting duct part 213 to the wound site W through the plurality of guide flow paths.

A first end of the drain tube 220 is connected to the first connecting duct part 212. Accordingly, the drain tube 220 may be placed in indirect communication with the sealed space S through the suction head 210. In some exemplary embodiments, the suction head 210 may not be provided, in which case, the first end of the drain tube 220 may be placed in direct communication with the sealed space S through the film dressing 120.

A second end of the drain tube 220 is connected to the canister 400. The canister 400 is connected to an output terminal 311 of a negative pressure generator 310, and thus, the drain tube 220 is placed in indirect communication with the negative pressure generator 310 through the canister 400.

Since the drain tube 220 is in communication with both the sealed space S and the negative pressure generator 310, the drain tube 220 may supply negative pressure generated by the negative pressure generator 310 to the sealed space S.

As illustrated in FIG. 1, the negative pressure generation unit 300 includes the negative pressure generator 310, a controller 320, which controls the negative pressure generator 310, and a pressure sensor 330, which senses negative pressure output from the negative pressure generator 310.

The negative pressure generation unit 300 includes the negative pressure generator 310 therein and supplies negative pressure generated by the negative pressure generator 310 to the negative pressure output unit 100 through the negative pressure delivery unit 200.

The negative pressure generator 310 generates negative pressure and supplies the negative pressure to the sealed space S through the drain tube 220. For example, the negative pressure generator 310 includes a negative pressure motor (not illustrated) therein and supplies negative pressure generated by the negative pressure motor to the sealed space S through the drain tube 220.

The operation of the negative pressure generator 310 is controlled by the controller 320. For example, the controller 320 may control whether and how long to operate the negative pressure motor and the magnitude of negative pressure to be generated by the negative pressure motor.

The negative pressure generated by the negative pressure generator 310 is provided to the drain tube 220 of the negative pressure delivery unit 200 through the output terminal 311. As illustrated in FIG. 1, the output terminal 311 is connected to a second side of the canister 400. Accordingly, the negative pressure generated by the negative pressure generator 310 is provided to the drain tube 220, which is connected to a first side of the canister 400, through the canister 400.

The pressure sensor 330 is connected to the output terminal 311 of the negative pressure generator 310.

The pressure sensor 330 senses the pressure in the sealed space S. For example, the pressure sensor 330 senses the pressure at the output terminal 311. Since the output terminal 311, the canister 400, the drain tube 220 of the negative pressure delivery unit 200, and the sealed space S form a connected space together, pressure information regarding the sealed space S may be obtained from pressure information sensed from the output terminal 311.

The controller 320 controls the negative pressure generator 310 and an irrigator 510 of the medication feeding unit 500. The controller 320 will be described later in detail.

Although not specifically illustrated in FIG. 1, the negative pressure generation unit 300 may also include a hydrophobic filter (not illustrated), which is provided between the canister 400 and the output terminal 311 of the negative pressure generator 310. The hydrophobic filter prevents a fluid such as exudate discharged from the sealed space S from infiltrating into the negative pressure generator 310.

Although not specifically illustrated in FIG. 1, the negative pressure generation unit 300 may also include a power button (not illustrated), which controls the turning on or off of the negative pressure generation unit 300. In response to the power button being turned on, an operation signal for operating the controller 320 is input to the controller 320. In response to the power button being turned off, an operation stop signal for stopping the operation of the negative pressure generator 310 is input to the controller 320.

Although not specifically illustrated in FIG. 1, the negative pressure generation unit 300 may also include a flow sensor (not illustrated), which measures the flow rate of a fluid discharged from the sealed space S due to negative pressure. The flow sensor may be disposed between the canister 400 and the negative pressure generator 310.

For example, the flow sensor may be connected to the output terminal 311 of the negative pressure generator 310. Accordingly, the flow sensor may measure the flow rate of a fluid flowing to the negative pressure generator 310 through the canister 400.

The fluid discharged from the sealed space S includes a gas and/or a liquid present in the sealed space S. The liquid present in the sealed space S may include exudate from the wound S.

The fluid discharged from the sealed space S flows into the canister 400 through the negative pressure delivery unit 200. Most of the liquid part of the fluid flowing into the canister 400 is contained in the canister 400 due to the gravity, and most of the gas part of the fluid flowing into the canister 400 flows to the negative pressure generator 310 due to negative pressure.

As a result, the flow sensor, which is disposed between the negative pressure delivery unit 200 and the negative pressure generator 310, can measure the flow rate of the gas present in the sealed space S, and flow rate information obtained by the flow sensor is transmitted to the controller 320.

The medication feeding unit 500 injects a medication into the wound W, which is sealed, and thus promotes the treatment of the wound W. The medication feeding unit 500 includes the irrigator 510, an oxygen supplier 520, and the connecting tube 530.

A medication to be injected into the wound site W may include water such as purified water or sterilized water. The medication may also include a treating agent promoting the treatment of the wound W. The medication may also include oxygen supplied by the oxygen supplier 520.

Once the medication is injected into the wound site W, the medication irrigates the wound W and is then discharged by negative pressure provided by the negative pressure generation unit 300. Accordingly, the medication may remove pathogens from the wound site W.

The irrigator 510 may contain a predetermined amount of the medication therein or may receive the medication from an additional container for storing the medication.

The irrigator 510 injects the medication into the wound site W. For example, the irrigator 510 may open an electronic valve (not illustrated), under the control of the controller 320, and may thus inject the medication into the wound site W. Also, the irrigator 510 may close the electronic valve, under the control of the controller 320, and may thus stop the injection of the medication into the wound site W.

In some exemplary embodiments, the irrigator 510 may include a pump (not illustrated). The irrigator 510 drives the pump, under the control of the controller 320, and may thus inject the medication into the wound site W. Also, the irrigator 510 may stop the driving of the pump and may thus stop the injection of the medication into the wound site W.

The irrigator 510 is connected to the connecting tube 530, which is connected to the second connecting duct part 213 of the suction head 210. Accordingly, the irrigator 510 may inject the medication into the wound site W through the connecting tube 530.

The irrigator 510 may inject the medication at a volume less than the volume of the wound site W.

The oxygen supplier 520 supplies oxygen to the medication to be fed to the wound site W. For example, the oxygen supplier 520 may be disposed above the connecting tube 530 and may supply oxygen to the medication flowing along the connecting tube 530.

The oxygen supplier 520 supplies oxygen to the medication only when the medication is fed to the wound site W under the control of the controller 320. For example, the oxygen supplier 520 may be driven at the time when the medication is injected, and may thus supply oxygen to the medication.

The connecting tube 530 connects the irrigator 510 and the second connecting duct part 213 of the suction head 210. Accordingly, the connecting tube 530 may deliver the medication of the irrigator 510 to the wound site W.

Since the connecting tube 530 of the medication feeding unit 500 is connected to the second connecting duct part 213 of the suction head 210, the irrigator 510 injects the medication into the wound site W through the suction head 210.

The medication feeding unit 500 is disposed in the sealed space S and also includes an additional injection head (not illustrated), which is connected to the connecting tube 530. The irrigator 510 injects the medication into the wound site W through the additional injection head.

In a case in which the negative pressure generation unit 300 and the irrigator 510 are provided as separate elements, as illustrated in FIG. 1, each of the negative pressure generation unit 300 and the irrigator 510 may include a communication part (not illustrated), which transmits control signals.

The communication parts of the negative pressure generation unit 300 and the irrigator 510 may be electrically connected by a wired cable (not illustrated) and may transmit control signals to, or receive control signals from, each other.

Although not specifically illustrated, each of the communication parts of the negative pressure generation unit 300 and the irrigator 510 may be configured to include a short-range wireless communication module such as a Bluetooth or Wireless Fidelity (WiFi) communication module and may thus be able to wirelessly transmit control signals to, or wirelessly receive control signals from, each other.

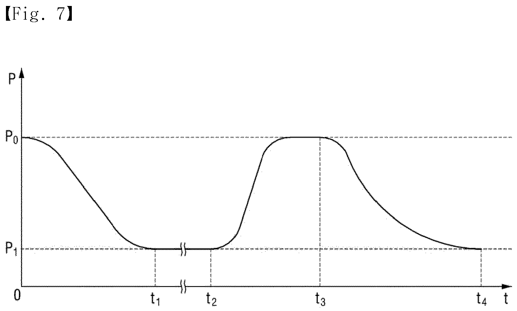

The controller 320 will hereinafter be described. The controller 320 may control the negative pressure generator 310 to lower the pressure in the sealed space S to a target negative pressure level (P₁ of FIG. 7).

Also, once the pressure in the sealed space S has reached the target negative pressure level, the controller 320 may control the negative pressure generator 310 to maintain the pressure in the sealed space S at the target negative pressure level (P₁ of FIG. 7) in a first setting period (from t₁ to t₂ of FIG. 7).

In a second setting period (from t₂ to t₃ of FIG. 7), which follows the first setting period (from t₁ to t₂ of FIG. 7), the controller 320 may control the negative pressure generator 310 to stop the supply of negative pressure to the sealed space S.

The controller 320 may control the irrigator 510 to inject the medication in the second setting period (from t₂ to t₃ of FIG. 7).

Also, the controller 320 may control the irrigator 510 to inject the medication into the wound site W at the time when the supply of negative pressure to the sealed space S is stopped. Since the supply of negative pressure is stopped and at the same time, the medication is injected into the wound site W, the medication may be easily injected into the wound site W due to the negative pressure in the sealed space S.

For example, when the supply of negative pressure to the sealed space S is stopped, the pressure in the sealed space S may be at the target negative pressure level (P₁ of FIG. 7), which is substantially a minimum pressure level, and may increase substantially to an atmospheric pressure level (P₀ of FIG. 7).

Accordingly, if the supply of negative pressure to the sealed space S is stopped and at the same time, the medication is injected into the wound site W, the medication may be quickly injected into the wound site W due to the pressure in the sealed space S.

Since the supply of negative pressure is stopped and at the same time, the medication is injected into the wound site W, the medication may be maintained to be in contact with the wound site W for a longer period of time than the second setting period (from t₂ to t₃ of FIG. 7).

Since the medication is maintained to be in contact with the wound site W for a predetermined amount of time, time may be secured for irrigating the wound site W to remove pathogens from the wound W. However, if the second setting period (from t₂ to t₃ of FIG. 7) is longer than necessary, the medication may be placed in contact with the wound site W for too long, so that the wound W may fester or granulation tissue from the wound W may be damaged.

Accordingly, the second setting period (from t₂ to t₃ of FIG. 7) may be set to be about 2 minutes, but the present disclosure is not limited thereto. That is, the length of the second setting period (from t₂ to t₃ of FIG. 7) may vary depending on the size and severity of a wound.

The first setting period (from t₁ to t₂ of FIG. 7) may be set to be longer than the second setting period (from t₂ to t₃ of FIG. 7). The supply of negative pressure to the sealed space S is mainly for discharging exudate from the wound site W. That is, the supply of negative pressure is basically for the treatment of the wound W. On the other hand, the injection of the medication into the wound site W is mainly for irrigating the wound site W so as to remove pathogens from the wound site W.

Accordingly, the first setting period (from t₁ to t₂ of FIG. 7), which corresponds to a wound treatment period, may be preferably set to be longer than the second setting period (from t₂ to t₃ of FIG. 7), which corresponds to a wound irrigation period. For example, the first setting period (from t₁ to t₂ of FIG. 7) may be set to about 13 minutes, and the second setting period (from t₂ to t₃ of FIG. 7) may be set to about 2 minutes.

The controller 320 may control the irrigator 510 to stop the injection of the medication into the wound site W in the second setting period (from t₂ to t₃ of FIG. 7). Accordingly, the injection of the medication into the wound site W and the discharge of the medication may be performed at different times.

The controller 320 may control the irrigator 510 to stop the injection of the medication into the wound site W after the second setting period (from t₂ to t₃ of FIG. 7). Accordingly, there may be an overlap in time between the injection of the injection of the medication into the wound site W and the discharge of the medication.

The controller 320 may determine whether the operation stop signal has been input by, for example, turning off the power button after the second setting period (from t₂ to t₃ of FIG. 7). In response to a determination being made that the operation stop signal has not yet been input, the controller 320 may control the negative pressure generator 310 to supply negative pressure to the sealed space S. In other words, in a case in which the operation stop signal has not yet been input, the controller 320 may drive the negative pressure generator 310 again.

Since the controller 320 supplies negative pressure to the sealed space S after the second setting period (from t₂ to t₃ of FIG. 7), the medication injected into the wound site W and a fluid such as exudate from the wound site W may be discharged into the canister 400.

The controller 320 controls the negative pressure generator 310 to lower the pressure in the sealed space S to the target negative pressure level (P₁ of FIG. 7) again.

The controller 320 generates control signals for controlling the irrigator 510. The control signals generated by the controller 320 may include a medication feed signal and a medication feed stop signal. The control signals generated by the controller 320 are transmitted to the irrigator 510 via the communication parts of the negative pressure generation unit 300 and the irrigator 510.

The controller 320 automatically calculates the volume of the wound site based on the flow rate of a flow, measured by the flow sensor (not illustrated). The volume of the wound site, calculated by the controller 320, may be used to determine the amount of injection of the medication into the wound site W.

For example, the controller 320 may calculate the volume of the wound site W by adding up the flow rate of a fluid discharged in the process of pressing the film dressing 120 and the foam dressing 110 against the surface of the wound site W.

Alternatively, the controller 320 may calculate the volume of the wound site by adding up the flow rate of a fluid measured from the time when the film dressing is pressed against the foam dressing 110 to the time when the pressure in the sealed space S reaches the target negative pressure level (P₁ of FIG. 7).

Alternatively, the controller 320 may calculate the volume of the wound site by adding up the flow rate of a fluid measured from the time when the film dressing is pressed against the foam dressing 110 to the time when the supply of negative pressure to the sealed space S is stopped.

Alternatively, the controller 320 may calculate the volume of the wound site by adding up the flow rate of a fluid measured from the time when the rate of change of the flow rate of the fluid exceeds a predetermined threshold level to the time when the pressure in the sealed space S reaches the target negative pressure level (P₁ of FIG. 7).

Once the volume of the wound site W is calculated, the controller 320 may automatically control the irrigator 510 to inject the medication at a volume less than the volume of the wound site W. For example, the controller 320 may control the amount of injection of the medication into the wound site W by controlling the electronic valve (not illustrated) or the pump (not illustrated) of the irrigator 510.

The controller 320 may control the irrigator 510 to stop the injection of the medication if the pressure in the sealed space S reaches the atmospheric pressure level (P₀ of FIG. 7) or a predetermined pressure level within the second setting period (from t₂ to t₃ of FIG. 7). Accordingly, the controller 320 may control the amount of injection of the medication according to the pressure in the sealed space S.

The controller 320 may control the oxygen supplier 520 to supply oxygen to the medication, which is to be injected into the wound site W. For example, the controller 320 may control the operation of the oxygen supplier 520 through the communication parts of the negative pressure generation unit 300 and the irrigator 510 so as for the oxygen supplier 520 to supply oxygen to the medication at the time when the medication is injected into the wound site W.

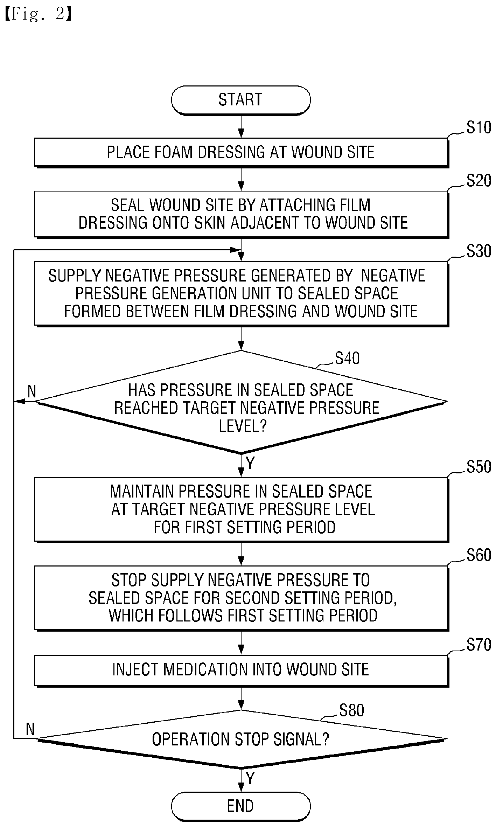

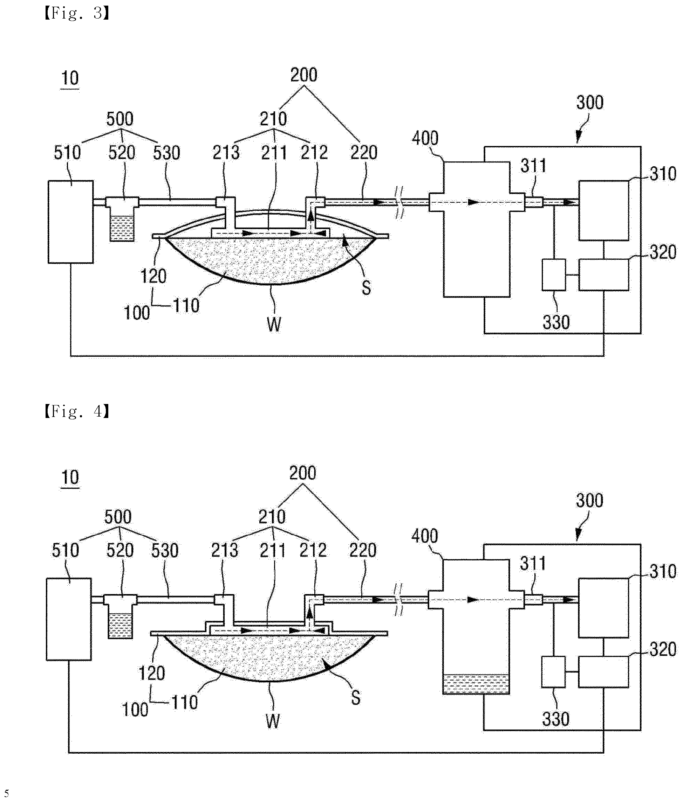

FIG. 2 is a flowchart illustrating an operating method of the wound treatment apparatus of FIG. 1. FIGS. 3 through 6 are schematic views illustrating how the wound treatment apparatus of FIG. 1 operates. FIG. 7 is a graph showing variations in the pressure in a sealed space during an operation of the wound treatment apparatus of FIG. 1.

Referring to FIGS. 2 through 7, a curer places the foam dressing 110 at the wound site W of a patient (S10). The foam dressing 110 placed at the wound site W absorbs exudate from the wound site W.

As illustrated in FIG. 3, the curer seals the wound site W (S20) by attaching the film dressing 120 onto the skin adjacent to the wound site W. As a result, the sealed space S is formed between the film dressing 120 and the wound site W, as illustrated in FIG. 1.

Once the wound site W is sealed, the curer supplies negative pressure to the sealed space S (S30) by driving the negative pressure generator 310 of FIG. 1. As a result, due to the negative pressure supplied by the negative pressure generator 310, the pressure in the sealed space S may be reduced from the atmospheric pressure level P₀ to the target negative pressure level P₁.

The controller 320 of FIG. 1 senses the pressure in the sealed space S using the pressure sensor 330 of FIG. 1, and determines whether the pressure in the sealed space S has reached the target negative pressure level P₁ (S40).

The target negative pressure level P₁ may be set to -125 mmHg or a range including -125 mmHg. For example, the target negative pressure level P₁ may be set to a range of ±5 mmHg from -125 mmHg.

The target negative pressure level P₁ of -125 mmHg may indicate a pressure level 125 mmHg lower than the atmospheric pressure. The atmospheric pressure is generally defined as about 760 mmHg, and the target negative pressure level P₁ may actually be about 635 mmHg, which is 125 mmHg lower than the atmospheric pressure. For convenience, the atmospheric pressure level P₀ may be defined as 0, and the target negative pressure level P₁ may be defined as -125 mmHg.

Once the pressure in the sealed space S has reached the target negative pressure level P₁, the controller 320 maintains the pressure in the sealed space S within a predetermined range from the target negative pressure level P₁ in the first setting period (from t₁ to t₂) (S50).

The first setting period (from t₁ to t₂ may be set to about 13 minutes, but may vary depending on the size and severity of a wound.

The controller 320 may uniformly maintain the pressure in the sealed space within a predetermined range by repeatedly turning on or off the negative pressure generator 310 in the first setting period (from t₁ to t₂).

For example, the controller 320 may repeatedly drive, and stop driving, the negative pressure generator 310 to maintain the pressure in the sealed space S at the target negative pressure level P₁. In other words, if the pressure in the sealed space S becomes higher than the target negative pressure level P₁, the controller 320 may drive the negative pressure generator 310 to lower the pressure in the sealed space S, and if the pressure in the sealed space S becomes lower than the target negative pressure level P₁, the controller 320 may stop driving the negative pressure generator 310 to increase the pressure in the sealed space S. Accordingly, the pressure in the sealed space S may be uniformly maintained at the target negative pressure level P₁.

If the pressure in the sealed space S has not reached the target negative pressure level P₁, the controller 320 may control the negative pressure generator 310 to supply negative pressure to the sealed space S and thus to allow the pressure in the sealed space S reach the target negative pressure level P₁.

Although not specifically illustrated in FIG. 2, the flow sensor (not illustrated) measures the flow rate of a fluid discharged from the sealed space S due to the negative pressure supplied by the negative pressure generation unit 300 to the sealed space S.

The flow sensor transmits the measured flow rate to the controller 320. The controller 320 may calculate the volume of the wound site W based on the measured flow rate transmitted by the flow sensor.

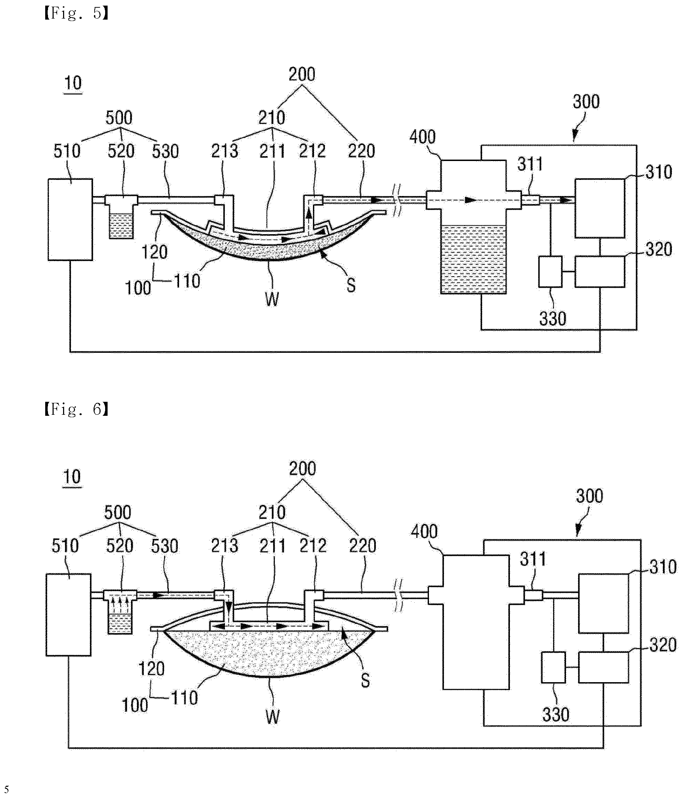

For example, as illustrated in FIG. 4, the controller 320 may continue to supply negative pressure to the sealed space S even after the film dressing 120 is pressed against the foam dressing 110. As a result, as illustrated in FIG. 5, the film dressing 120 and the foam dressing 110 may be pressed against the surface of the wound site W. That is, the foam dressing 110 with the film dressing 120 pressed thereagainst may be pressed against the surface of the wound site W.

A fluid in the sealed space S may be discharged in the process of pressing the film dressing 120 and the foam dressing 110 against the surface of the wound site W. Since the foam dressing 110 is placed at the wound site W, the amount of a fluid discharged in the process of pressing the film dressing 120 and the foam dressing 110 against the surface of the wound site W may substantially correspond to the volume of the wound site W.

Accordingly, the controller 320 may calculate the volume of the wound site W by adding up the amount of a fluid discharged in the process of pressing the film dressing 120 and the foam dressing 110 against the surface of the wound site W.

Alternatively, as illustrated in FIG. 3, in the process of pressing the film dressing 120 against the foam dressing 110, the tensile force of the film dressing 130 may act as counterforce to negative pressure.

On the other hand, as illustrated in FIGS. 4 and 5, in the process of pressing the film dressing 120 and the foam dressing 110 against the surface of the wound site W, the tensile force of the film dressing 130 and the compressive force of the foam dressing 110 may both act as counterforce to negative pressure.

Since the counterforce to negative pressure in the process of pressing the film dressing 120 and the foam dressing 110 against the surface of the wound site W is stronger than the counterforce to negative pressure in the process of pressing the film dressing 120 against the foam dressing 110, the amount of a fluid discharged in the in the process of pressing the film dressing 120 and the foam dressing 110 against the surface of the wound site W may be less than the amount of a fluid discharged in the in the process of pressing the film dressing 120 against the foam dressing 110. Thus, the flow rate of a fluid discharged from the sealed space S may considerably change.

Accordingly, the controller 320 may calculate the volume of the wound site W by adding up the amount of a fluid measured from the time when the rate of change of the flow rate of a fluid measured by the flow sensor exceeds a predetermined threshold level.

For example, the controller 320 may calculate the volume of the wound site W by adding up the flow rate of a fluid measured from the time when the time when the rate of change of the flow rate of a fluid exceeds the predetermined threshold level to the time when the pressure in the sealed space S reaches the target negative pressure level P₁.

Alternatively, the controller 320 may calculate the volume of the wound site W by adding up the flow rate of a fluid measured from the time when the time when the rate of change of the flow rate of a fluid exceeds the predetermined threshold level to the time when the driving of the negative pressure generator 310 is stopped (i.e., the flow rate of a fluid measured during the first setting period after the pressure in the sealed space S has reached the target negative pressure level P₁).

In the second setting period (from t₂ to t₃), which follows the first setting period (from t₁ to t₂), the controller 320 stops the supply of negative pressure generated by the negative pressure generator 310 to the sealed space S (S60).

The second setting period (from t₂ to t₃) may be set to about 2 minutes, but may vary depending on the size and severity of a wound.

As illustrated in FIG. 6, the controller 320 controls the irrigator 510 of FIG. 1 to inject the medication into the wound site W within the second setting period (from t₂ to t₃) (S70).

The controller 320 may control the irrigator 510 to inject the medicine into the wound site W at the time when the supply of negative pressure to the sealed space S is stopped. Accordingly, the efficiency of the treatment of a wound may be improved by leaving no interval of time between the time when the supply of negative pressure to the sealed space S is stopped and the time when the medication is injected into the wound site W.

If the supply of negative pressure is stopped and at the same time, the injection of the medication is injected, the medication may be maintained to be in contact with the wound site W for a longer period of time than the second setting period (from t₂ to t₃).

Also, the controller 320 may control the irrigator 510 to inject the medication at a volume less than the volume of the wound site W.

After the second setting period (from t₂ to t₃), the controller 320 determines whether the operation stop signal for stopping the operation of the negative pressure generator 310 has been input (S80).

In response to a determination being made that the operation stop signal has not yet been input, the controller 320 drives the negative pressure generator 310 again to supply negative pressure to the sealed space S. That is, after the second setting period (from t₂ to t₃), the controller 320 may repeatedly perform the step of lowering the pressure in the sealed space S to the target negative pressure level P₁ until the operation stop signal is input.

Since negative pressure is supplied again to the sealed space S, the medication injected into the wound site W may be discharged into the canister 400. The controller 320 may control the irrigator 510 to stop the injection of the medication into the wound site W during the second setting period (from t₂ to t₃), in which case, the injection of the medication and the discharge of the medication may occur at different times.

However, as already mentioned above, if the controller 320 controls the irrigator 510 to stop the injection of the medication into the wound site W after the second setting period (from t₂ to t₃), there may be an overlap in time between the injection of the medication and the discharge of the medication.

In some exemplary embodiments, a cycle of steps ranging from the supply of negative pressure to the sealed space to the injection of the medication into the wound site W may be repeatedly performed a predetermined number of times, and then, the operation of the negative pressure generation unit 300 may be stopped even if no operation stop signal is input.

In concluding the detailed description, those skilled in the art will appreciate that many variations and modifications can be made to the preferred embodiments without substantially departing from the principles of the present invention as defined by the claims. Therefore, the disclosed preferred embodiments of the invention are used in a generic and descriptive sense only and not for purposes of limitation.

## Claims

1. A wound treatment apparatus (10), comprising:
a foam dressing (110) configured to be placed at a wound site (W);
an irrigator (510) configured to feed a medication to the wound site (W);
a film dressing (120) configured to be attached onto the skin adjacent to the wound site (W) so as to seal the wound site (W);
a negative pressure generation unit (310) configured to generate negative pressure and to supply the negative pressure to a sealed space (S), which is formed between the film dressing (120) and the wound site (W);
a negative pressure delivery unit (200) configured to connect the negative pressure generation unit (310) and the sealed space (S) and to deliver the negative pressure to the sealed space (S); and
a controller (320) configured to control the negative pressure generation unit (310) and the irrigator (510),
wherein the controller (320) is configured to control the negative pressure generation unit (310) and the irrigator (510) such that the supply of the negative pressure is stopped and at the same time, the medication is injected into the wound site (W),
**characterized in that**
the wound treatment apparatus (10) further comprises
a flow sensor configured to measure a flow rate of a fluid discharged from the sealed space (S) due to the negative pressure,
wherein the controller (320) is configured to calculate the volume of the wound site (W) based on the flow rate measured by the flow sensor, and
once the volume of the wound site (W) is calculated, the controller (320) is configured to control the irrigator (510) to inject the medication at a volume less than the volume of the wound site (W).

2. The wound treatment apparatus (10) of claim 1, wherein once the pressure in the sealed space (S) reaches the target negative pressure level, the controller (320) is configured to control the negative pressure generation unit (310) to maintain the pressure in the sealed space (S) at the target negative pressure level for a first setting period.

3. The wound treatment apparatus (10) of claim 2, wherein the controller (320) is configured to control the negative pressure generation unit (310) to stop the supply of the negative pressure for a second setting period, which follows the first setting period, and to control the irrigator (510) to inject the medication at a time when the supply of the negative pressure is stopped.

4. The wound treatment apparatus (10) of claim 3, wherein the controller (320) is configured to control the irrigator (510) to stop the injection of the medication within the second setting period.

5. The wound treatment apparatus (10) of claim 3, wherein the controller (320) is configured to control the irrigator (510) to stop the injection of the medication after the second setting period.

6. The wound treatment apparatus (10) of claim 3, wherein the first setting period is longer than the second setting period.

7. The wound treatment apparatus (10) of claim 1, further comprising:
an oxygen supplier (520) configured to supply oxygen to the medication, which is to be injected into the wound site (W).

## Patentansprüche

1. Wundbehandlungsvorrichtung (10), mit:
einem Schaumverband (110), der ausgestaltet ist, an einer Wundstelle (W) platziert zu werden,
einem Rieseler (510), der ausgestaltet ist, der Wundstelle (W) ein Arzneimittel zuzuführen,
einem Filmverband (120), der ausgestaltet ist, benachbart zu der Wundstelle (W) an der Haut angebracht zu werden, um die Wundstelle (W) zu versiegeln,
einer Unterdruckerzeugungseinheit (310), die ausgestaltet ist, Unterdruck zu erzeugen und den Unterdruck einem versiegelten Raum (S) zuzuführen, der zwischen dem Filmverband (120) und der Wundstelle (W) gebildet ist,
einer Unterdruckliefereinheit (200), die ausgestaltet ist, die Unterdruckerzeugungseinheit (310) und den versiegelten Raum (S) zu verbinden und den Unterdruck an den versiegelten Raum (S) zu liefern, und
einer Steuereinheit (320), die ausgestaltet ist, die Unterdruckerzeugungseinheit (310) und den Rieseler (510) zu steuern,
wobei die Steuereinheit (320) ausgestaltet ist, die Unterdruckerzeugungseinheit (310) und den Rieseler (510) so zu steuern, dass die Zufuhr von Unterdruck unterbrochen wird und zur gleichen Zeit das Arzneimittel in die Wundstelle (W) injiziert wird,
**gekennzeichnet dadurch, dass**
die Wundbehandlungsvorrichtung (10) ferner umfasst:
einen Durchflusssensor, der ausgestaltet ist, eine Durchflussrate eines Fluids zu messen, das infolge des Unterdrucks aus dem versiegelten Raum (S) abgeführt wird,
wobei die Steuereinheit (320) ausgestaltet ist, das Volumen der Wundstelle (W) auf Basis der durch den Flusssensor gemessenen Durchflussrate zu berechnen, und
sobald das Volumen der Wundstelle (W) berechnet ist, die Steuereinheit (320) ausgestaltet ist, den Rieseler (510) zu steuern, um das Arzneimittel mit einem Volumen geringer als dem Volumen der Wundstelle (W) zu injizieren.

2. Wundbehandlungsvorrichtung (10) nach Anspruch 1, wobei, sobald der Druck in dem versiegelten Raum (S) das Zielunterdruckniveau erreicht, die Steuereinheit (320) ausgestaltet ist, die Unterdruckerzeugungseinheit (310) zu steuern, um den Druck in dem versiegelten Raum (S) für eine erste Einstellzeitdauer an dem Zielunterdruckniveau zu halten.

3. Wundbehandlungsvorrichtung (10) nach Anspruch 2, wobei die Steuereinheit (320) ausgestaltet ist, die Unterdruckerzeugungseinheit (310) zu steuern, um die Zufuhr von Unterdruck für eine zweite Einstellzeitdauer zu unterbrechen, die auf die erste Einstellzeitdauer folgt, und den Rieseler (510) zu steuern, um das Arzneimittel zu einem Zeitpunkt zu injizieren, bei dem die Zufuhr des Unterdrucks beendet ist.

4. Wundbehandlungsvorrichtung (10) nach Anspruch 3, wobei die Steuereinheit (320) ausgestaltet ist, den Rieseler (510) zu steuern, um die Injektion des Arzneimittels innerhalb der zweiten Einstellzeitdauer zu beenden.

5. Wundbehandlungsvorrichtung (10) nach Anspruch 3, wobei die Steuereinheit (320) ausgestaltet ist, den Rieseler (510) zu steuern, um die Injektion des Arzneimittels nach der zweiten Einstellzeitdauer zu beenden.

6. Wundbehandlungsvorrichtung (10) nach Anspruch 3, wobei die erste Einstellzeitdauer länger als die zweite Einstellzeitdauer ist.

7. Wundbehandlungsvorrichtung (10) nach Anspruch 1, ferner mit:
einer Sauerstoffzufuhr (520), die ausgestaltet ist, dem Arzneimittel, das in die Wundstelle (5) zu injizieren ist, Sauerstoff zuzuführen.

## Revendications

1. Appareil de traitement de plaie (10), comprenant :
un pansement hydrocellulaire (110) configuré pour être placé au niveau d'un site de plaie (W) ;
un irrigateur (510) configuré pour approvisionner un médicament au site de plaie (W) ;
un pansement film (120) configuré pour être attaché sur la peau adjacente au site de plaie (W) pour ainsi sceller le site de plaie (W) ;
une unité de génération de pression négative (310) configurée pour générer une pression négative et pour fournir la pression négative à un espace scellé (S), qui est formé entre le pansement film (120) et le site de plaie (W) ;
une unité de délivrance de pression négative (200) configurée pour raccorder l'unité de génération de pression négative (310) et l'espace scellé (S) et pour délivrer la pression négative à l'espace scellé (S) ; et
un dispositif de commande (320) configuré pour commander l'unité de génération de pression négative (310) et l'irrigateur (510),
dans lequel le dispositif de commande (320) est configuré pour commander l'unité de génération de pression négative (310) et l'irrigateur (510) de telle sorte que la fourniture de la pression négative est arrêtée et en même temps, le médicament est injecté dans le site de plaie (W),
**caractérisé en ce que**
l'appareil de traitement de plaie (10) comprend en outre
un détecteur d'écoulement configuré pour mesurer un débit d'un fluide déchargé de l'espace scellé (S) en raison de la pression négative,
dans lequel le dispositif de commande (320) est configuré pour calculer le volume du site de plaie (W) sur la base du débit mesuré par le détecteur d'écoulement, et
une fois que le volume du site de plaie (W) est calculé, le dispositif de commande (320) est configuré pour commander à l'irrigateur (510) d'injecter le médicament à un volume inférieur au volume du site de plaie (W).

2. Appareil de traitement de plaie (10) selon la revendication 1, dans lequel une fois que la pression dans l'espace scellé (S) atteint le niveau de pression négative cible, le dispositif de commande (320) est configuré pour commander à l'unité de génération de pression négative (310) de maintenir la pression dans l'espace scellé (S) au niveau de pression négative cible pendant une première période de réglage.

3. Appareil de traitement de plaie (10) selon la revendication 2, dans lequel le dispositif de commande (320) est configuré pour commander à l'unité de génération de pression négative (310) d'arrêter la fourniture de la pression négative pendant une seconde période de réglage, qui suit la première période de réglage, et pour commander à l'irrigateur (510) d'injecter le médicament à un moment où la fourniture de pression négative est arrêtée.

4. Appareil de traitement de plaie (10) selon la revendication 3, dans lequel le dispositif de commande (320) est configuré pour commander à l'irrigateur (510) d'arrêter l'injection du médicament pendant la deuxième période de réglage.

5. Appareil de traitement de plaie (10) selon la revendication 3, dans lequel le dispositif de commande (320) est configuré pour commander à l'irrigateur (510) d'arrêter l'injection du médicament après la deuxième période de réglage.

6. Appareil de traitement de plaie (10) selon la revendication 3, dans lequel la première période de réglage est plus longue que la deuxième période de réglage.

7. Appareil de traitement de plaie (10) selon la revendication 1, comprenant en outre :
un dispositif de fourniture d'oxygène (520) configuré pour fournir de l'oxygène au médicament, qui doit être injecté dans le site de plaie (W).
